(19) Europäisches Patentamt
European Patent Office
Office européen des brevets

(11) **EP 4 628 153 B1**

(12) **EUROPEAN PATENT SPECIFICATION**

(45) Date of publication and mention
of the grant of the patent:
**29.04.2026 Bulletin 2026/18**

(21) Application number: **25160567.1**

(22) Date of filing: **27.02.2025**

(51) International Patent Classification (IPC):
*A61N 1/37* (2006.01)    *A61N 1/08* (2006.01)
*A61B 5/0245* (2006.01)    *A61B 5/308* (2021.01)
*A61B 5/00* (2006.01)

(52) Cooperative Patent Classification (CPC):
**A61N 1/3718; A61B 5/0245; A61B 5/308;**
**A61B 5/686; A61B 5/7221; A61N 1/08**

(54) **IMPLANTABLE MEDICAL DEVICE DETECTION OF NON-BIOLOGICAL DISTURBANCE**

NACHWEIS NICHTBIOLOGISCHER STÖRUNGEN DURCH IMPLANTIERBARE MEDIZINISCHE VORRICHTUNG

DÉTECTION DE PERTURBATION NON BIOLOGIQUE D'UN DISPOSITIF MÉDICAL IMPLANTABLE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **03.04.2024  US 202463574106 P**
**25.02.2025  US 202519062852**

(43) Date of publication of application:
**08.10.2025  Bulletin 2025/41**

(60) Divisional application:
**26161340.0 / 4 725 404**

(73) Proprietor: **Pacesetter, Inc.**
**Sylmar, CA 91342 (US)**

(72) Inventors:
• **Andersen, Dean**
**Sylmar, CA 91342 (US)**
• **Shirai, Eiji**
**Sylmar, CA 91342 (US)**

(74) Representative: **Barker Brettell Sweden AB**
**Kungsbroplan 3**
**112 27 Stockholm (SE)**

(56) References cited:
EP-B1- 2 207 593    US-A- 5 810 739
US-A- 5 910 156    US-A1- 2006 173 295
US-A1- 2013 253 352    US-A1- 2016 114 169
US-A1- 2017 042 482    US-A1- 2022 111 201

## Description

### FIELD OF TECHNOLOGY

Embodiments of the present technology described herein generally relate to implantable medical devices (IMDs) that include, or are electrically coupled to, electrodes that are intended to be in contact with tissue of patients within which the IMDs are implanted, e.g., for the purpose of sensing an electrocardiogram (ECG) or an electrogram (EGM) that is indicative of cardiac electrical activity.

### BACKGROUND

Various types of IMDs include or are electrically coupled to electrodes that are intended to be in contact with tissue of the patients within which the IMDs are implanted. For example, an insertable cardiac monitor (ICM) is a type of IMD that is implanted in a pocket of tissue under a patient's skin such that a pair of electrodes of the ICM are in contact with tissue of the patient and can be used to sense an electrocardiogram (ECG) to enable continuous monitoring of the patient's cardiac electrical activity. In such an ICM, one electrode that is used to sense the ECG can be an electrically conductive housing of the ICM, while another electrode that is used to sense the ECG can be within a header of the ICM. For another example, an implantable cardioverter defibrillator (ICD) and/or a pacemaker can include or be electrically coupled to one or more cardiac leads, on which are located electrodes that are placed in contact with cardiac tissue. With such an IMD, two or more electrodes can be used to sense an electrogram (EGM) to enable monitoring of the patient's cardiac electrical activity. Additionally, two or more electrodes of such an IMD may be used to deliver pacing therapy and/or other types of cardiac therapy, such as cardioversion shocks and/or anti-tachycardia pacing, but not limited thereto.

After an ICM is implanted within a pocket of tissue within a patient, it is possible that at least one of the electrodes of the ICM may temporarily or permanently lose contact with tissue of the patient, e.g., due to movement of the ICM within the pocket and/or due to maturation of the pocket. When this happens, abrupt changes occur to the ECG signal being sensed by the ICM, which may cause undesirable false detections of various types of cardiac events or episodes. Similarly, after a cardiac lead is implanted such that its electrodes are in contact with cardiac tissue, it is possible that at least one of the electrodes of the cardiac lead may temporarily or permanently lose contact with the cardiac tissue. When this happens, abrupt changes occur to the EGM signal being sensed by the ICD and/or pacemaker to which the cardiac lead is coupled, which may cause undesirable false detections of various types of cardiac events or episodes. Abrupt changes to the EGM signal being sensed by the ICD and/or pacemaker may also occur if the cardiac lead becomes defective over time, which similarly may cause undesirable false detections of various types of cardiac events or episodes.

US 2013/253352 A1 discloses a method for detecting potential failures by an implantable medical lead. The method includes sensing first, second and third signals between at least first and second combinations of electrodes, on the lead; determining whether at least one of the first, second and third signals is representative of a potential failure in the lead and identifies a failure and the electrode associated with the failure based on which of the first, second and third sensed signals is representative of the potential failure.

EP 2207593 B1 relates to detecting overload within an analog-to-digital converter (ADC) of an IMD. The IMD includes an overload detection module that determines whether the ADC is operating in an overload condition. When the overload detection module determines the ADC is operating in the overload condition for a particular period of time, the ADC sends an overload signal to a processor that processes the output of the ADC. The overload signal notifies the processor that the ADC is operating in or is close to operating in the overload condition. In response to the indication from the ADC, the processor of the IMD disregards the output of the ADC. The processor continues to disregard the output of the ADC until the overload signal is deactivated, thereby indicating that the ADC is no longer in an overloaded condition.

US 5910156 A discloses method and apparatus for detection of non-physiologic senses checks the output of the sense amplifier of an IMD where the inputs of the sense amplifier are adapted to connect to a living body for sensing electrical signals representative of physiologic events, and if that sense output senses a physiologic event within a predetermined time after a non-physiologic event is sensed by another circuit also connected to receive electrical signals from said living body, then there is reported out a signal indicating that the event sensed by the sense amplifier is of a non-physiologic nature.

US 2016/114169 A1 discloses an intracardiac pacemaker configured to receive a cardiac electrical signal developed across a pair of electrodes coupled to the pacemaker and detect a crossing of a first sensing threshold of the cardiac electrical signal. A pacing escape interval timer is set to a first pacing escape interval in response to the cardiac electrical signal crossing the first sensing threshold. The pacing escape interval timer is adjusted if the cardiac electrical signal crosses a second sensing threshold during a time limit.

US 5810739 A discloses methods and apparatus for classifying cardiac events with an implantable cardiac device. Cardiac signals are stored in a buffer. When a significant cardiac event is confirmed, a corresponding segment of the stored cardiac signals is processed to generate a set of feature values. The set of feature values is compared to various sets of reference values. Each set of reference values preferably corresponds to a separate type of cardiac event, so that the cardiac device may classify the significant cardiac event by matching its set of feature values to a set of reference values. Feature values include the maximum positive and negative slew rates exhibited by the cardiac signal during the segment, the maximum positive and negative signal amplitudes, the times to reach the maximum positive and negative signal amplitudes, the area under the cardiac signal curve, the area under the cardiac signal curve above zero as compared to the area below zero, and the number of zero crossings made by the cardiac signal. Additional feature values are generated by simultaneously processing the cardiac signals measured along two different vector directions in the heart.

US 2022/111201 A1 discloses a system and/or method involving sensing first data via at least one implantable sensor of an IMD system, and identifying a presence-absence state of a magnetic resonance imaging (MRI) system using the first data.

US 2006/173295 A1 discloses a medical device for providing therapy to a host patient comprises a stimulation engine for generating a stimulation regimen and configured to provide the regimen to the patient in a first mode of operation, and a detector coupled to the stimulation engine for detecting the presence of a strong magnetic field and placing the stimulation engine in a second mode of operation in response thereto.

US 2017/042482 A1 discloses an IMD including a sensing module configured to receive a cardiac electrical signal via electrodes carried by a medical electrical lead coupled to the implantable medical device and a control module configured to detect a lead issue. The sensing module is configured to produce cardiac sensed event signals and spike detect signals. The control module is configured to determine event intervals defined by consecutive ones of the received cardiac sensed event signals and the received spike detect signals and identify one or more received spike detect signals as lead issue spikes based on at least one of the determined event intervals.

SUMMARY

The present invention is defined in the independent claim. Further embodiments are defined in the dependent claims.

Certain embodiments of the present technology are related to an insertable cardiac monitor (ICM) configured to be subcutaneously implanted in a patient, wherein the ICM comprises first and second electrodes intended to be in contact with tissue of the patient within which the ICM is subcutaneously implanted. The ICM also comprises sense circuitry and disturbance detection circuitry. The sense circuitry is configured to produce a differential signal indicative of a voltage potential difference between first and second electrodes. The disturbance detection circuitry is configured to detect when at least one of the first or the second electrodes of the ICM loses contact with the tissue of the patient within which the ICM is subcutaneously implanted based at least in part on a slew rate of the differential signal exceeding a slew rate threshold.

In accordance with certain embodiments, the disturbance detection circuitry is configured to detect when the at least one of the first or the second electrodes loses contact with the tissue of the patient within which the ICM is subcutaneously implanted also based on a magnitude of the differential signal exceeding a magnitude threshold for at least a threshold period of time.

In accordance with certain embodiments, the differential signal comprises one of an electrocardiogram (ECG) indicative of cardiac electrical activity of the patient within which the ICM is subcutaneously implanted.

In accordance with certain embodiments, the IMD also comprises a controller that is communicatively coupled to the disturbance detection circuitry. The controller can be configured to detect an episode of asystole or sinus pause, and classify the episode of asystole or sinus pause as being a false positive when the detection that at least one of the first or the second electrodes loses contact with the tissue of the patient detected by the disturbance detection circuitry at least partially coincides with the episode of asystole or sinus pause. Alternatively, or additionally, the controller can be configured to detect a premature ventricular contraction (PVC) or premature atrial contraction (PAC), and classify the detected PVC or PAC as being a false detection when the detection that at least one of the first or the second electrodes loses contact with the tissue of the patient detected by the disturbance detection circuitry at least partially coincides with the detected PVC or PAC. Additionally, or alternatively, the controller can be configured to issue a warning or a notification in response to the the disturbance detection circuitry detecting that at least one of the first or the second electrodes loses contact with the tissue of the patient within which the ICM is subcutaneously implanted.

In accordance with certain embodiments, the sense circuitry comprises an amplifier having differential inputs electrically coupled to the first and the second electrodes and an analog-to-digital converter (ADC) configured to digitize an output of the amplifier.

In accordance with certain embodiments, the disturbance detection circuitry comprises: a digital differentiator configured to differentiate the output of the ADC; a first digital comparator configured to compare an output of the digital differentiator to a positive slew rate threshold; a second digital comparator configured to compare the output of the digital differentiator to a negative slew rate threshold; and logic (e.g., an OR gate or a portion of a controller of software of firmware thereof) configured to receive outputs of the first and the second digital comparators and provide an indication of when a slew rate of the output of the ADC is greater than the positive slew rate threshold or less than the negative slew rate threshold.

In accordance with certain embodiments, the disturbance detection circuitry further comprises: a third digital comparator configured to compare an output of the ADC to a positive amplitude threshold; a fourth digital comparator configured to compare the output of the ADC to a negative amplitude threshold; and further logic (e.g., a further OR gate or a further portion of the controller or software or firmware thereof) configured to receive outputs of the third and the fourth digital comparators and provide an indication of when an amplitude of the output of the ADC is greater than the positive amplitude threshold or less than the negative amplitude threshold.

In accordance with certain embodiments, the disturbance detection circuitry further comprises a counter configured to receive an output of the further logic and periodically increment a value of the counter while the output of the ADC remains greater than the positive amplitude threshold, or the output of the ADC remains less than the negative amplitude threshold.

In accordance with certain embodiments, the sense circuitry comprises an amplifier having differential inputs electrically coupled to the first and the second electrodes, the disturbance detection circuitry comprises analog circuitry configured to detect when the slew rate of the differential signal exceeds the slew rate threshold, and the slew rate threshold is specified based on a potential difference between a pair of reference voltages and a period of a signal that is used to control switches of a pair of sample and hold circuits of the analog circuitry.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

## BRIEF DESCRIPTION OF THE DRAWINGS

Embodiments of the present technology relating to both structure and method of operation may best be understood by referring to the following description and accompanying drawings, in which similar reference characters denote similar elements throughout the several views:

FIG. 1 is a high level block diagram that illustrates how an IMD can use a pair of electrodes to sense a differential signal, which can be an ECG or an EGM, but is not limited thereto

FIG. 2A illustrates an embodiment of the present technology that can be incorporated into an IMD to detect fast slew rates in an ECG or EGM (or other differential signal) that should only occur in response to a non-biological disturbance, such as an electrode losing contact with patient tissue, or the IMD being exposed to EMI.

FIG. 2B illustrates another embodiment of the present technology that can be incorporated into an IMD to detect fast slew rates in an ECG or EGM (or other differential signal) that should only occur in response to a non-biological disturbance, such as an electrode losing contact with patient tissue, or the IMD being exposed to EMI.

FIG 3 illustrates results of a simulation of an embodiment of the present technology.

FIGS. 4A and 4B are high level flow diagrams that are used to summarize methods according to various embodiments of the present technology that can be used by an IMD to detect a non-biological disturbance, such as an electrode losing contact with patient tissue, or the IMD being exposed to EMI.

FIG. 5 shows a block diagram of one embodiment of an IMD with which an embodiment of the present technology can be used to detect a non-biological disturbance.

## DETAILED DESCRIPTION

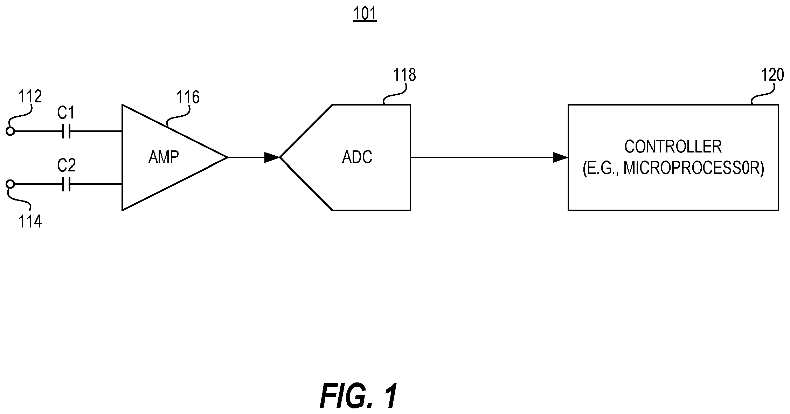

FIG. 1 is a high level block diagram that illustrates how an IMD 101 can use a pair of electrodes 112, 114 to sense a differential signal, which can be an ECG or an EGM, but is not limited thereto. The electrodes 112, 114 can be part of the IMD 101, or can be electrically coupled to the IMD 101. Shown in FIG. 1 is a sense amplifier 116 having its differential inputs electrically coupled to the pair of electrodes 112, 114. The optional capacitors C1 and C2, which are within the signal paths between the electrodes 112, 114 and differential inputs of the sense amplifier 116, are DC blocking capacitors. Where the IMD is an ICM, there may be no need for switches between the electrodes 112, 114 and the sense amplifier 116. However, where the IMD is an ICD and/or pacemaker, there may be switches (not shown) between more than two electrodes and the sense amplifier 116, and the switches may be controlled to enable various different pairs of the electrodes to be coupled to the differential inputs of the sense amplifier 116, as is known in the art.

Still referring to FIG. 1, while the electrodes 112, 114 are in contact with patient tissue, the sense amplifier 116 outputs a differential signal indicative of a voltage potential difference between the electrodes 112, 114. Such a differential signal, as noted above, can be an ECG or an EGM, but is not limited thereto. As also noted above, if the first and second electrodes are implanted within or on a patient's brain tissue, the differential signal (indicative of the voltage potential difference between the first and the second electrodes) can be a brain signal, e.g., a deep brain signal. If the first and second electrodes are implanted in or on tissue of, or proximate to, a patient's spinal cord, the differential signal (indicative of the voltage potential difference between the first and the second electrodes) can be a spinal signal. These are just a few examples of the various types of differential signals that can be sensed, and based upon which, an embodiment of the present technology can be used to sense a non-biological disturbance. These examples are not intended to be all encompassing, and one of ordinary skill in the art reading this disclosure will appreciate that embodiments of the present technology can be used with alternative types of different signals indicative of a voltage potential difference between first and second electrodes. The differential signal (e.g., an ECG or EGM) that is output by the sense amplifier 116 is shown as being digitized, i.e., converted from an analog signal to a digital signal, by an analog to digital converter (ADC) 118, and the digitized differential signal (e.g., digitized ECG or EGM) is shown as being provided to a controller 120. The controller 120, which can be implemented, for instance, by a processor and/or state machine, but is not limited thereto, can analyze the digitized differential signal (e.g., digitized ECG or EGM) to detect various cardiac events, such R-waves corresponding to ventricular depolarizations, P-waves corresponding to atrial depolarizations, premature ventricular contractions (PVCs), premature atrial contractions (PACs), and/or the like. The controller 120 can also detect various types of cardiac episodes, such as episodes of ventricular tachycardia (VT), atrial tachycardia (AT), ventricular fibrillation (VF), atrial fibrillation (AF), asystole, sinus pause, and/or the like.

While not specifically shown in FIG. 1, it is possible that one or more analog filters can be located upstream and/or downstream of the sense amplifier 116. Additionally, or alternatively, it is possible that one or more digital filters can be located downstream of the ADC 118. It is also possible that one or more additional amplifiers can be located upstream and/or downstream of the sense amplifier 116. It is also possible that analog R-wave detection circuitry can be located within the signal path between the sense amplifier 116 and the ADC 118, or that digital R-wave detection circuitry be located within the signal path between the ADC 118 and the controller 120, as is known in the art. More generally, it should be understood that additional and/or alternative circuitry can be included to provide filtering, amplifying, and/or detections of one or more types of cardiac events, but not limited thereto, as would be appreciated by one of skill in the art.

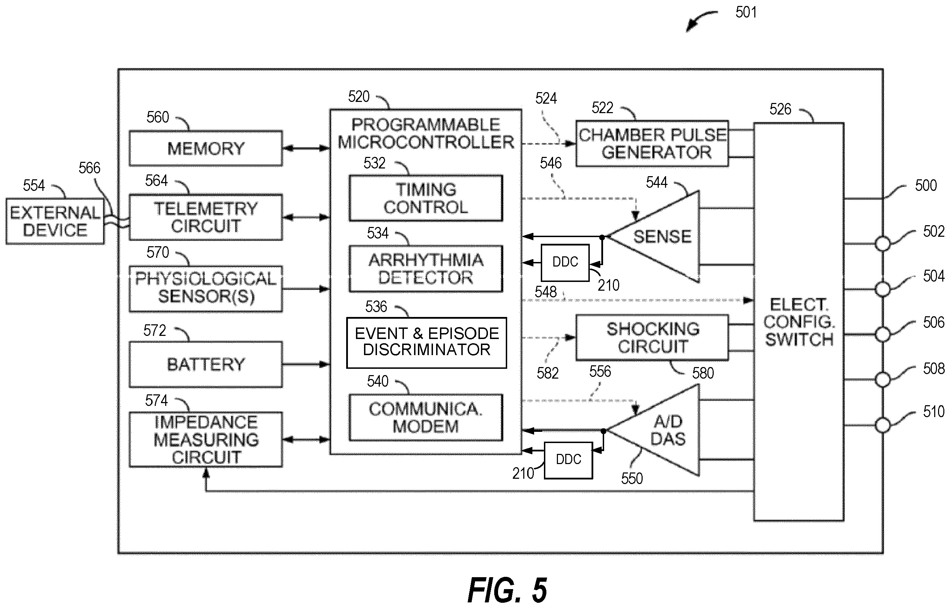

While not specifically shown in FIG. 1, the IMD 101 may include various other components and/or modules, such as memory, a telemetry circuit, physiologic sensor(s), a battery, an impedance measure circuit, and/or the like, e.g., as can be appreciated from the below discussion of the example IMD 501 described below with reference to FIG. 5. If the IMD 101 is capable of delivering therapy, then the IMD 101 may include pulse generator(s) and/or a shocking circuit, and/or the like, e.g., as can be appreciated from the below discussion of the example IMD 501 described below with reference to FIG. 5.

Still referring to FIG. 1, if one of the electrodes 112, 114 loses contact with patient tissue, a large DC offset between the electrodes 112, 114 occurs due to there being a large voltage, on the order of a half-cell potential, between the electrodes 112, 114. This large voltage difference is amplified by the sense amplifier 116. A gain of the sense amplifier 116 can be, e.g., 100 V/V, but is not limited thereto. Because of the gain, the large voltage difference between the inputs of the sense amplifier 116 will cause the output of the sense amplifier 116 to saturate at one of its supply voltage rails, e.g., +/- 1.8 Volts, but not limited thereto. The saturation will occur substantially immediately, limited only by a bandwidth of the sense amplifier 116, which creates a very fast change in the differential signal output by the sense amplifier 116.

As will be appreciated from the below description, in accordance with certain embodiments of the present technology, the above described very fast change in the differential signal can be detected by monitoring the slew rate of the differential signal (e.g., an ECG or EGM) that is output by the sense amplifier 116.

Slew rate (SR) is defined as the change in a signal per unit of time. Mathematically this can be expressed using the following equation:

$$SR = max \left| \frac{dV}{dt} \right|.$$

The maximum slew rate is the difference between a present signal and a previous signal. By definition, this is a differentiation operation. In differential equations, this can be expressed using the following equation:

$$y[n] = x[n] - x[n-1]$$

where

> $y[n]$ is the present output of the differentiator operation for a sample n,
> $x[n]$ is the present output of the ADC 118 that is operated on by the differentiator operation, and
> $x[n-1]$ is the previous output of the ADC 118 that is used by the differential operation.

When one of the electrodes 112, 114 loses contact with patient tissue, two things happen at the output of the sense amplifier 116, and subsequently, at the output of the ADC 118. Explained another way, when one of the electrodes 112, 114 loses contact with patient tissue, two things happen to the sensed differential signal (e.g., an ECG or EGM) output by the sense amplifier 116, and subsequently, to the digitized version thereof output by the ADC 118. The sensed signal output by the sense amplifier 116 (and the digitized version thereof output by the ADC 118) saturates at one polarity (e.g., at +/- 1.8 Volts) and the output signal of the sense amplifier 116 (and the output of the ADC 118) has a very high slew rate as it goes to saturation.

The slew rate of the differential signal (e.g., an ECG or EGM) that is output by the sense amplifier 116, which will be very fast when one of the electrodes 112, 114 loses contact with patient tissue, is limited by the bandwidth of the sense amplifier 116, as noted above, and in the digital domain is limited by the response time of the ADC 118. Typically, the very fast slew rate that occurs when one of the electrodes 112, 114 loses contact with patient tissue is on order of hundreds of millivolts per second (mV/s).

By contrast, a typical ECG or EGM does not have slew rates that ever come close to the slew rates that occur when one of the electrodes 112, 114 loses contact with patient tissue. For example, an R-wave peak time, which is a time from an onset of the R-wave to a time of a peak of the R-wave, is typically in the range of about 15 milliseconds (ms) to 35 ms. Even if an R-wave peak time is very short in duration, e.g., about 1 millisecond (ms), the slew rate would still be relatively slow, e.g., within the range of about 20 mV/sec to 70 mV/sec. Certain embodiments of the present technology take advantage of this distinction between naturally occurring slew rates and slew rates that occur when one of the electrode 112, 114 loses contact with patient tissue (as well as when some other types of non-biological disturbances occur) to distinguish between naturally occurring cardiac events or episodes (such as R-waves, P-waves, PVCs, PACs, episodes of asystole, episodes of sinus pause, but not limited thereto) and non-biological disturbances (such as one of the electrodes 112, 114 losing contact with patient tissue, or an IMD being exposed to electromagnetic interference (EMI), but not limited thereto).

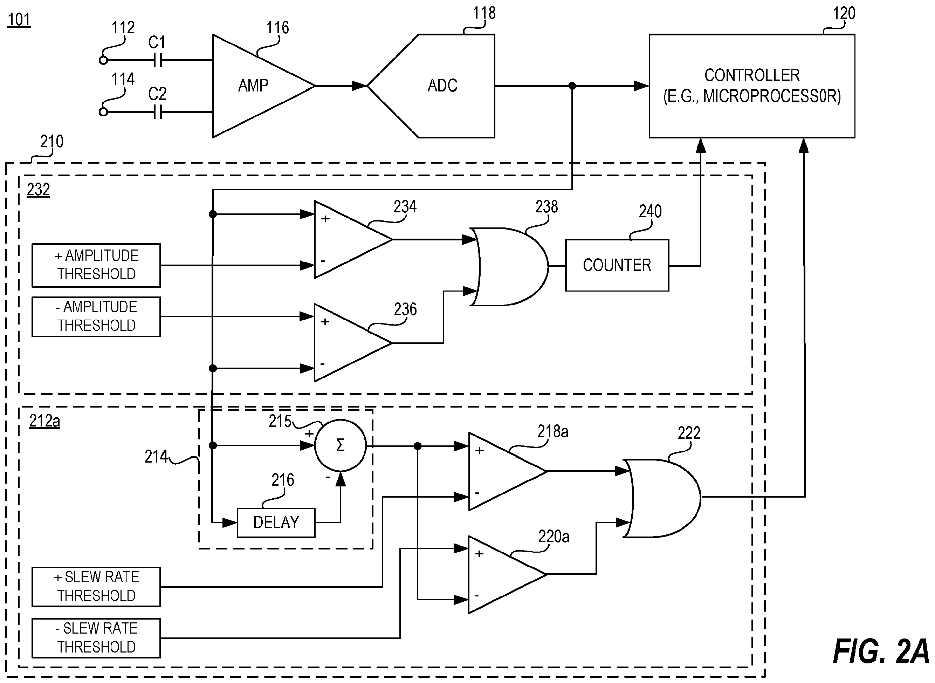

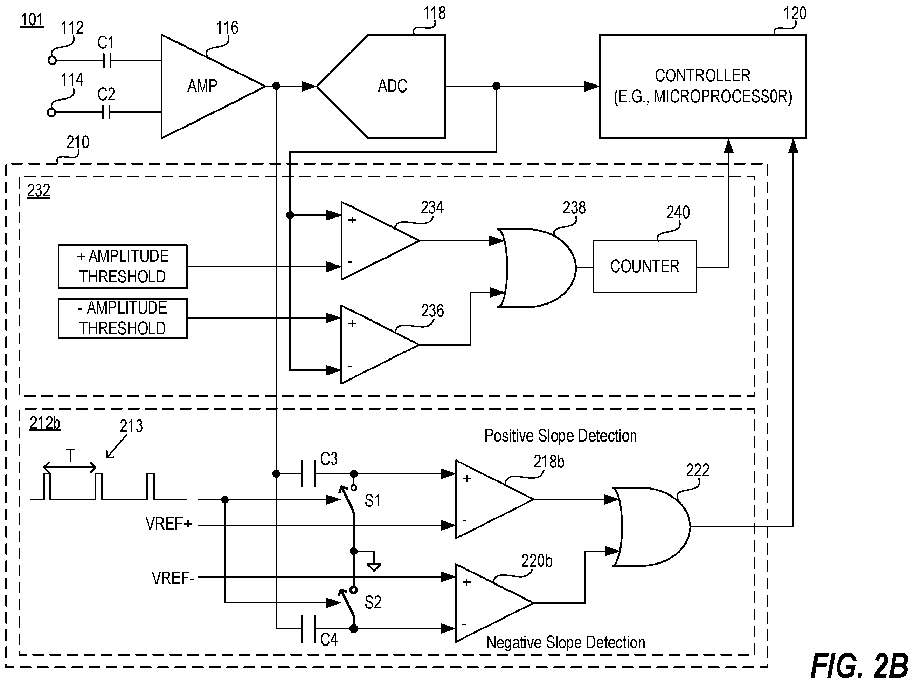

The high level block diagrams of FIGS. 2A and 2B will now be used to illustrate examples of embodiments of the present technology that can detect fast slew rates in an ECG or EGM (or other type of differential signal) that should only occur in response to non-biological disturbances, such as one of the electrode 112, 114 losing contact with patient tissue, or an IMD being exposed to EMI. Referring to FIG. 2A, shown therein is non-biological disturbance detection circuitry (DDC) 210, which includes fast slew rate detection circuitry 212a and optionally also includes further circuitry 232. The fast slew rate detection circuitry 212a is configured to detect, in the digital domain, when a slew rate of the differential signal output by the sense amplifier 116 (after the differential signal has been digitized by the ADC 118) exceeds a specified slew rate threshold. The fast slew rate detection circuitry 212a includes differentiator circuitry 214, digital comparators 218a and 220a, and an OR gate 222. The fast slew rate detection circuitry 212a (and 212b described below) can also be referred to herein as the fast slew rate detector 212a (or 212b).

The differentiator circuitry 214 can perform the differentiator operation described above. The differentiator circuitry 214 is shown as including a delay register 216 and a summer 215 (and more specifically, a subtractor 215) that is configured to determine a difference between a digitized output and a previous digitized output of the ADC 118 (e.g., an $n^{th}$ value output by the ADC 118 minus an $n-1^{th}$ value (i.e., previous) output by the ADC 118). The difference value output by the summer 215 (and more generally, output by the differentiator circuitry 214), which corresponds to the slew rate of the digitized version of the differential signal (e.g., the ECG or EGM) that is output by the ADC 118, is preferably provided to the both a positive input of the comparator 218a, and to a negative input of the comparator 220a. A negative input of the comparator 218a is provided with a positive slew rate threshold value, and a positive input of the comparator 220a is provide with a negative slew rate threshold value, as shown. In this configuration, the output of the comparator 218a will transition from low to high (from binary 0 to binary 1) when the slew rate of the differential signal output by the sense amplifier 116 (and more specifically, the digitized version thereof output by the ADC 118) has a slew rate that exceeds the positive slew rate threshold value, e.g., which may be caused by the electrode 114 losing contact with patient tissue. The output of the comparator 220a will transition from low to high (from binary 0 to binary 1) when the slew rate of the differential signal output by the sense amplifier 116 (and more specifically, the digitized version thereof output by the ADC 118) has a slew rate that exceeds the negative slew rate threshold value, e.g., which may be caused by the electrode 112 losing contact with patient tissue. By contrast, while the electrodes 112 and 114 remain in contact with patient tissue, the slew rates of the differential signal output by the sense amplifier 116 (and more specifically, the digitized version thereof output by the ADC 118) should be relatively slow and should not exceed either one of the positive or negative slew rate thresholds, and thus, the outputs of both comparators 218a and 220a should remain low (have a binary 0 value).

The outputs of the comparators 218a and 220a are provided to inputs of the OR gate 222, such that when at least one of the electrodes 112 or 114 loses contact with patient tissue (or some other type of non-biological disturbance resulting a fast slew rate is detected), the output of the OR gate 222 will transition from low to high (from binary 0 to binary 1). The output of the OR gate 222 is shown as being provided to a controller 120 (e.g., microprocessor) of the IMD 101, so that the controller 120 is informed of the detection of the fast slew rate (by the fast slew rate detection circuitry 212), which may have been caused by at least one of the electrodes 112 or 114 losing contact with patient tissue, or by some other type of non-biological disturbance, such as the IMD being exposed to EMI. The controller 120 can use this information in various manners. For example, the controller 120 can use this information to classify a detected episode of asystole or sinus pause as being a false positive. In other words, the controller 120 can use the information from the fast slew rate detection circuitry 212a to distinguish between a true episode of asystole or sinus pause (that was detected in response to R-waves not being detected for at least a respective threshold period of time due to the absence of ventricular contractions) and a false detection of asystole or sinus pause (that was detected in response to R-waves not being detected for at least a respective threshold period of time due to a non-biological disturbance, such as at least one of the electrodes 112 or 114 losing contact with patient tissue, or the IMD being exposed to EMI). The controller 120 can also be used to distinguish between actual and false detections of PVCs, PACs, and/or the like. Further, it is noted that the logic of the OR gate 222 can be implemented by the controller 120.

At least one of the electrodes 112 or 114 losing contact with patient tissue is one possible type of non-biological disturbance that an IMD 101 can detect based at least in part on whether the slew rate of a differential signal (e.g., an ECG or EGM) exceeds a slew rate threshold. Another type of non-biological disturbance that an IMD 101 can detect based at least in part on whether the slew rate of a differential signal (e.g., an ECG or EGM) exceeds a slew rate threshold is the IMD 101 being exposed to EMI that causes the sense amplifier 116 to saturate. It is also possible that other types of non-biological disturbances that cause fast slew rates in the sensed differential signal (e.g., ECG or EGM) can be detected using an embodiment of the present technology. Such another type of non-biological disturbance includes exposure to a time-varying gradient magnetic field from a magnetic resonance imaging (MRI) system.

It is possible that a relatively brief (transient) non-biological disturbance can cause the fast slew rate detection circuitry 212a to inform the controller 120 of a fast slew rate being detected, e.g., if one of the electrodes 112 or 114 loses contact with patient tissue for a relative brief period of time (e.g., one second), or if the IMD 101 is exposed to EMI for a relative brief period of time (e.g., one second). It is also possible that a relatively brief (transient) biological disturbance can cause the fast slew rate detection circuitry 212a to inform the controller 120 of a fast slew rate being detected, e.g., due to a large amplitude R-wave or PVC briefly saturating the sense amplifier 116. In order to distinguish between a relatively brief (transient) non-biological or biological disturbance and a relatively long lasting non-biological disturbance, the further circuitry 232 can be used to determine whether a magnitude of the differential signal (e.g., the ECG or EGM) exceeds a magnitude threshold (e.g., set just below the rail voltage, but not limited thereto) for at least a threshold period of time (e.g., at least four seconds, but not limited thereto). Using such further circuitry 232, the controller 120 can detect the non-biological disturbance also based on the magnitude of the differential signal exceeding the magnitude threshold for at least the threshold period of time (e.g., four seconds, but not limited thereto). The further circuitry 232 can also be referred to herein as the saturation detector 232.

Still referring to FIG. 2A, shown therein is an example implementation of the further circuitry 232 configured to determine whether a magnitude of the differential signal (e.g., the ECG or EGM) exceeds a magnitude threshold (e.g., set just below the rail voltage, but not limited thereto) for at least a threshold period of time (e.g., four seconds, or ten seconds, but not limited thereto). The circuitry 232 includes digital comparators 234 and 236, an OR gate 238, and a counter 240. A negative input of the comparator 234 is provided with a positive amplitude threshold value, and a positive input of the comparator 236 is provide with a negative amplitude threshold value, as shown. In certain embodiments, the positive amplitude threshold value and the negative amplitude threshold have the same absolute value (e.g., 1.7 V), but have different polarities (e.g., + 1.7 V and - 1.7 V), so that they collectively define the magnitude threshold (e.g., 1.7 V). It is also within in the scope of the embodiments described herein that an absolute value of the positive amplitude threshold value differs from an absolute value of the negative amplitude threshold.

In the configuration shown in FIG. 2A, the output of the comparator 234 will transition from low to high (from binary 0 to binary 1) when the amplitude the differential signal output by the sense amplifier (and more specifically, the digitized version thereof output by the ADC 118) has an amplitude that exceeds the positive amplitude threshold value, e.g., which may be caused by the electrode 114 losing contact with patient tissue. The output of the comparator 236 will transition from low to high (from binary 0 to binary 1) when the amplitude of the differential signal output by the sense amplifier 116 (and more specifically, the digitized version thereof output by the ADC 118) falls below (i.e., is more negative than) the negative amplitude threshold value, e.g., which may be cause by the electrode 112 losing contact with patient tissue. By contrast, while the electrodes 112 and 114 remain in contact with patient tissue, the amplitude of the differential signal output by the sense amplifier 116 (and the digitized version thereof output by the ADC 118) should remain within the range specified by the positive and negative amplitude thresholds, and thus, the outputs of both comparators 234 and 236 should remain low (have a binary 0 value). In some embodiments, the sense amplifier 116 is adjusted to cause the differential signal to substantially remain within a specified range.

The outputs of the comparators 234 and 236 are provided to inputs of the OR gate 238, such that when at least one of the electrodes 112 or 114 loses contact with patient tissue, the output of the OR gate 238 will transition from low to high (from binary 0 to binary 1). The output of the OR gate 238 is provided to the counter 240. The counter 240 is configured to produce a count value indicative of how long the output of the OR gate 238 remains high and the output of the counter 240 is provided to the controller 120. In this manner, the controller 120 can monitor the length (duration) of time that a disturbance is detected, to thereby enable the controller 120 to distinguish between a relatively brief (transient) non-biological disturbance that is detected (e.g., due to one of the electrodes 112 or 114 losing contact with patient tissue for just one second, or the IMD 101 being exposed to EMI for just one second) and a longer lasting non-biological disturbance (e.g., due to one of the electrodes 112 or 114 losing contact with patient tissue for at least four seconds, or the IMD 101 being exposed to EMI for at least four seconds). Monitoring the length of time that a disturbance is detected also enables the controller 120 to distinguish between a relatively brief (transient) biological disturbance that is detected (e.g., due to a large amplitude R-wave or PVC briefly saturating the sense amplifier 116) and a longer lasting non-biological disturbance (e.g., due to one of the electrodes 112 or 114 losing contact with patient tissue for at least four seconds, or the IMD 101 being exposed to EMI for at least four seconds). Depending upon the specific implementation, the counter 240 can be reset automatically whenever the input to the counter 240 (which is the output of the OR gate 238) transitions from high to low, or by the controller 120. It would also be possible to eliminate the counter 240 and provide the output of the OR gate 238 directly to the controller 120 and the controller 120 itself can monitor how long the output of the OR gate 238 remains high. One of skill in the art reading this description would understand that other variations to implement the circuitry described with reference to FIG. 2A are also possible that are within the scope of the embodiments of the present technology described herein. For example, the logic of the OR gate 238 and the counting performed by the counter 240 can be implemented by the controller 120. For another example, rather than implementing a fast slew rate detector or detection circuitry 212a within the digital domain, as was the case in FIG. 2A, the fast slew rate detector or detection circuitry 212b can instead be implemented in the analog domain, as described below with reference to FIG. 2B.

Referring to FIG. 2B, shown therein is non-biological disturbance detection circuitry (DDC) 210, which includes fast slew rate detection circuitry 212b and optionally also includes the further circuitry 232. The fast slew rate detection circuitry 212b is configured to detect, in the analog domain, when a slew rate of the differential signal output by the sense amplifier 116 exceeds a specified slew rate threshold.

The fast slew rate detector 212b is shown as including switches S1 and S2, capacitors C3 and C4, analog comparators 218b and 220b, and an OR gate 222. The switch S1 and the capacitor C3 are configured as a sample and hold circuit, wherein the capacitor C3 is repeatedly sampled and holds the output voltage of the sense amplifier 116 at a rate that is responsive to the timing signal 213 that controls the switch S1 and has a period T. Similarly, the switch S2 and the capacitor C4 are configured as a sample and hold circuit, wherein the capacitor C4 is repeatedly sampled and holds the output voltage of the sense amplifier 116 at a rate that is responsive to the timing signal 213 that controls the switch S2 and has the period T. More specifically, as the switches S1 and S2 are turned on and off (closed and opened), the capacitors C3 and C4 are sampled and hold the output of the sense amplifier 116 at a rate specified by the period T of the timing signal 213 that controls the switches S1 and S2. The voltage stored on the capacitor C3 at any given time is provided to the non-inverting (+) input of the comparator 218b, and the voltage stored on the capacitor C4 at any given time is provided to the inverting (-) input of the comparator 220b. A positive reference voltage ($V_{REF+}$) is provided to the inverting (-) input of the comparator 218b, and a negative reference voltage ($V_{REF-}$) is provided to the non-inverting (+) input of the comparator 220b. A slew rate threshold of the fast slew rate detector 212b is specified in accordance with the equation dV/dt threshold = ($V_{REF+}$ - GND)/T for the comparator 218b and in accordance with the equation dV/dt threshold = ($V_{REF-}$ - GND)/T for the comparator 220b, such that whenever a slew rate of the differential signal (e.g., ECG or EGM) that is output by the sense amplifier 116 exceeds the specified slew rate threshold, the output of one of the comparators 218b or 220b will transition from low to high (from binary 0 to 1), causing the output of the OR gate 222 to similarly transition from low to high (from binary 0 to 1) thereby providing an indication of a fast slew rate detection to the controller 120. More specifically, when the slew rate of a positive sloped portion of the differential signal (e.g., ECG or EGM) output by the sense amplifier 116 exceeds the specified slew rate threshold the output of the comparator 218b will transition from low to high (from binary 0 to 1), and when the slew rate of a negative sloped portion of the differential signal (e.g., ECG or EGM) output by the sense amplifier 116 exceeds the specified slew rate threshold the output of the comparator 220b will transition from low to high (from binary 0 to 1).

Still referring to FIG. 2B, shown therein is also the further circuitry 232 configured to determine whether a magnitude of the differential signal (e.g., the ECG or EGM) exceeds a magnitude threshold (e.g., set just below the rail voltage, but not limited thereto) for at least a threshold period of time (e.g., four seconds, or ten seconds, but not limited thereto). The further circuitry 232 operates in the same manner as was described above with reference to FIG. 2A, and thus need not be described again. As was also the case in FIG. 2A, the further circuitry 232 in FIG. 2B is implemented in the digital domain, and analyzes the differential signal (ECG or EGM) output by the sense amplifier 116 after the signal has been digitized by the ADC 118. In alternative embodiments, further circuitry configured to determine whether a magnitude of the differential signal (e.g., the ECG or EGM) exceeds a magnitude threshold (e.g., set just below the rail voltage, but not limited thereto) for at least a threshold period of time (e.g., four seconds, or ten seconds, but not limited thereto) can be implemented in the analog domain.

In accordance with certain embodiments, the controller 120 in FIGS. 1, 2A, and 2B (or the controller 520 in FIG. 5) can be configured to issue a warning or a notification when one of the first or the second electrodes 112, 114 loses contact with the tissue of the patient within which the IMD 101 is implanted. The warning or notification can be auditory, vibratory, and/or stored in memory of the IMD 101. The warning or notification can alternatively, or additionally, be communicated from the IMD 101 to an external device (e.g., external device 554 in FIG. 5) that can store information about the warning or notification, can display the warning or notification, and/or can otherwise inform a patient and/or physician about the warning or notification.

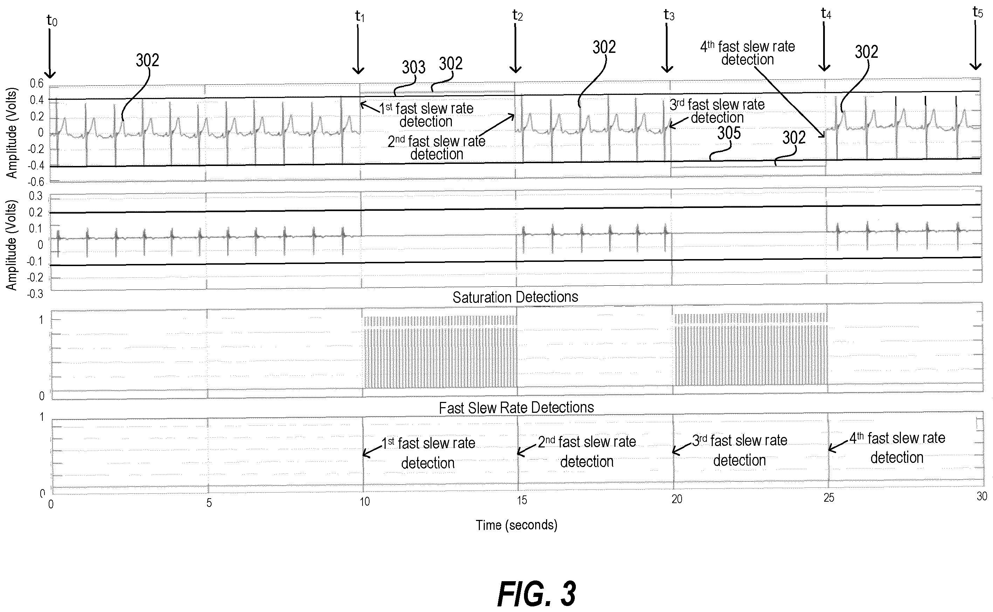

FIG. 3 illustrates results of a simulation of an embodiment of the present technology. The first (i.e., uppermost) panel in FIG. 3 shows an example differential signal 302 output by the sense amplifier 116 (after it has been digitized by the ADC 118), which differential signal can also be referred to herein as the sensed signal. The second panel in FIG. 3 shows an output of a differentiator function, e.g., provided by the differentiator circuitry 214. The third panel in FIG. 3 shows saturation detections that correspond to clock counts. The fourth (i.e., lowermost) panel in FIG. 3 shows the high slew rate detections. During the time period between 0 seconds and 10 seconds (labeled $t_0$ and $t_1$ respectively) the electrodes 112 and 114 are in contact with patient tissue and the sensed signal output by the sense amplifier 116 resembles a typical EGM signal from which cardiac events, such as ventricular depolarizations, can be identified. At 10 seconds (labeled $t_1$) the electrode 112 is disconnected from patient tissue (i.e., loses contact with patient tissue), which causes a first fast slew rate detection, and several saturations are detected spaced 200 ms apart (corresponding to the counter being incremented every 200 ms, while the electrode 112 remains disconnected from patient tissue). During an entirety of the time period between 10 seconds and 15 seconds (labeled $t_1$ and $t_2$ respectively), while the electrode 112 remains disconnected from patient tissue, a magnitude of the differential signal 302 output by the sense amplifier 116 exceeds a positive amplitude threshold 303, and more generally exceeds the magnitude threshold. During this period of time, the differential signal saturates to a high voltage rail of the sense amplifier 116, which in this example is +0.5 V.

At 15 second (labeled $t_2$) the electrode 112 is reconnected to patient tissue (i.e., is placed back in contact with patient tissue), which causes the sensed signal output by the sense amplifier 116 to again resemble a typical EGM signal from which cardiac events, such as ventricular depolarizations, can be identified. At 15 second (labeled $t_2$), in response to the electrode 112 being reconnected to patient tissue (i.e., being placed back in contact with patient tissue), a second fast slew rate detection occurs. During a majority of the time period between 15 seconds and 20 seconds (labeled $t_2$ and $t_3$ respectively), while the electrode 112 remains connected from patient tissue, a magnitude of the differential signal 304 output by the sense amplifier 116 is below the positive amplitude threshold 303, and more generally remains below the magnitude threshold.

At 20 seconds (labeled $t_3$) the other electrode 114 is disconnected from patient tissue (i.e., loses contact with patient tissue), which causes a third fast slew rate detection, and several saturations are detected spaced 200 ms apart (corresponding to the counter being incremented every 200 ms, while the electrode 114 remains disconnected from patient tissue). During an entirety of the time period between 20 seconds and 25 seconds (labeled $t_3$ and $t_4$ respectively), while the electrode 114 remains disconnected from patient tissue, a magnitude of the differential signal 302 output by the sense amplifier 116 remains below a negative amplitude threshold 305, and more generally exceeds the magnitude threshold. During this period of time, the differential signal saturates to a low voltage rail of the sense amplifier 116, which in this example is -0.5 V.

At 25 second (labeled $t_4$) the electrode 114 is reconnected to patient tissue (i.e., is placed back in contact with patient tissue), which causes the sensed signal output by the sense amplifier 116 to again resemble a typical EGM signal from which cardiac events, such as ventricular depolarizations, can be identified. At 25 second (labeled $t_4$), in response to the electrode 114 being reconnected to patient tissue (i.e., being placed back in contact with patient tissue), a fourth fast slew rate detection occurs. During a majority of the time period between 20 seconds and 25 seconds (labeled $t_4$ and $t_5$ respectively), while the electrode 112 remains connected from patient tissue, a magnitude of the differential signal 304 output by the sense amplifier 116 is below the positive amplitude threshold 303, and more generally remains below the magnitude threshold.

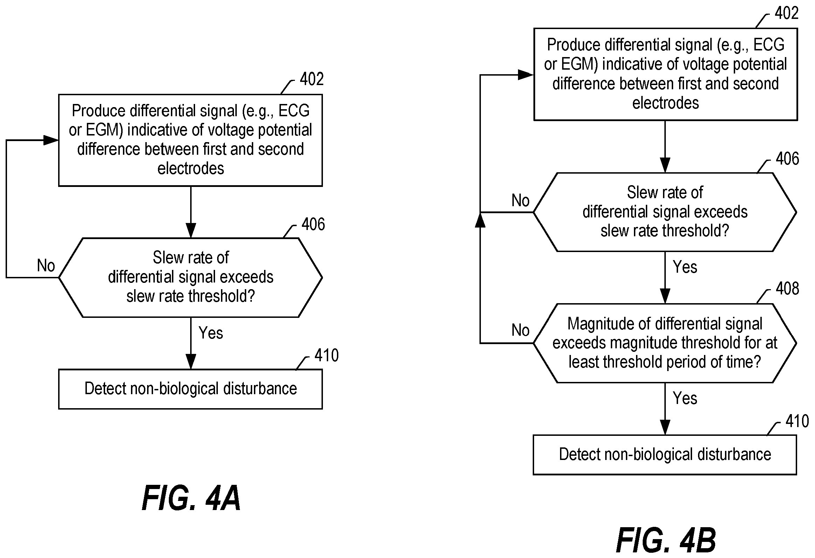

FIGS. 4A and 4B are high level diagrams that are used to summarize methods according to certain embodiments of the present technology. Such methods are for use with an IMD (e.g. 101 or 501) implanted in a patient, wherein the IMD includes first and second electrodes (e.g., 112 and 114) that are intended to be in contact with tissue of the patient within which the IMD is implanted. The steps of such methods can be performed by and/or under the control of a controller (e.g., 120 or 520) of an IMD.

Referring initially to FIG. 4A, step 402 involves producing (e.g., sensing) a differential signal indicative of a voltage potential difference between the first and the second electrodes. Step 402 can be performed, e.g., using a sense amplifier (e.g., 116) that includes differential inputs that are electrically coupled to the first and the second electrodes (e.g., 112 and 114), e.g., via one or more switches and/or via one or more DC blocking capacitors (e.g., C1 and C2). Still referring to FIG. 4A, step 406 involves determining whether a slew rate of the differential signal exceeds a slew rate threshold. Step 406 can be performed, e.g., using the fast slew rate detection circuitry 212a or 212b described above with reference to FIGS. 2A and 2B, but is not limited thereto. If the answer to the determination at step 406 is No, then flow returns to step 402. If the answer to the determination at step 406 is Yes, then a non-biological disturbance is detected at step 410. The method of FIG. 4B is similar to the method of FIG. 4A, but includes an additional step 408 that is used to distinguish between a relatively brief (transient) non-biological disturbance and one that is longer lasting. Steps 402 and 406 in FIG. 4B are the same as in FIG. 4A, and, thus, need not be described again. If the answer to the determination at step 406 is Yes in the method of FIG. 4B, then flow goes to step 408. At step 408 there is a determination of whether a magnitude of the differential signal exceeds a magnitude threshold for at least a threshold period of time. Step 408 can be performed, e.g., using the further circuitry 232 described above with reference to FIGS. 2A and 2B. If the answer to the determination at step 408 is No, then flow returns to step 402. If the answer to the determination at step 408 is Yes, then a non-biological disturbance is detected at step 410. It is also noted that steps 406 and 408 can be performed simultaneously such that when the answer to both determinations are Yes, a non-biological disturbance is detected.

In accordance with certain embodiments, the slew rate threshold (e.g., used at step 406) can be within a range from 100 mV/sec to 500 mV/sec (e.g., 200 mV/sec), but is not limited thereto. In accordance with certain embodiments, a value for the magnitude threshold (e.g., used at step 408) depends on expected peak values of the sensed differential signal (e.g., the EGM or ECG signal) indicative of the voltage potential difference between first and second electrodes. For example, the magnitude threshold can be a value within a range of about 1.4 to 2.0 times (e.g., 1.5 times) the expected peak values. In accordance with certain embodiments, the threshold period of time (e.g., used at step 408) can be within a range of 100 msec to 200 msec (e.g., 128 msec), but is not limited thereto.

A non-biological disturbance can be detected at step 410 in response to at least one of the first electrode (e.g., 112) or the second electrode (e.g., 114) losing contact with the tissue of the patient within which the IMD is implanted. Alternatively, a non-biological disturbance can be detected at step 410 in response to an IMD including (or electrically coupled to) the first electrode (e.g., 112) and the second electrode (e.g., 114) being exposed to electromagnetic interference (EMI). Alternatively, a non-biological disturbance can be detected at step 410 in response to an IMD including (or electrically coupled to) the first electrode (e.g., 112) and the second electrode (e.g., 114) being exposed to a time-varying gradient magnetic field from an MRI system. It is also possible that the methods summarized above with reference to FIGS. 4A and 4B can be used to detect one or more other types of non-biological disturbances that cause a fast slew rate in the sensed signal and cause saturation of a sensing amplifier having differential signals electrically coupled to the first and second electrodes.

Such a method can be used to determine whether to classify a detected episode of asystole or sinus pause as being a false detection. For example, if a non-biological disturbance at least partially coincides with or in close temporal proximity with a detected episode of asystole or sinus pause, then the detected episode of asystole or sinus pause can be classified as a false positive. Additionally, or alternatively, such a method can be used to determine whether to classify a detected PVC or a detected PAC as being a false detection. For example, if a non-biological disturbance at least partially coincides with or in close temporal proximity with a detected PVC or a detected PAC, then the detected PVC or PAC can be classified as a false positive.

Such a method can additionally, or alternatively, be used to issue a warning or a notification in response to a non-biological disturbance being detected at step 410. As noted above, the warning or notification can be auditory, vibratory, and/or stored in memory of the IMD. The warning or notification can alternatively, or additionally, be communicated from the IMD to an external device (e.g., 554) that can store information about the warning or notification, can display the warning or notification, and/or can otherwise inform a patient and/or physician about the warning or notification.

In accordance with certain embodiments, step 408 can be performed by comparing an amplitude of the differential signal to a positive amplitude threshold and to a negative amplitude threshold. In response the amplitude of the differential signal being greater than the positive amplitude threshold, or being less than the negative amplitude threshold, there can be the determination that the magnitude of the differential signal exceeds the magnitude threshold. In certain such embodiments, a value of a counter (e.g., 240) is periodically incremented while the amplitude of the differential signal remains greater than the positive amplitude threshold, or remains less than the negative amplitude threshold. In response the value of the counter being equal to or greater than a counter threshold, there can be the determination that the magnitude of the differential signal exceeds the magnitude threshold for at least the threshold period of time.

As was described above with reference to FIG. 2A, the determination of whether the slew rate of the differential signal exceeds the slew rate threshold, which is performed at step 406, can be performed in a digital domain using a digital differentiator circuit. As was described above with reference to FIG. 2B, the determination of whether the slew rate of the differential signal exceeds the slew rate threshold can alternatively be performed in an analog domain.

FIG. 5 shows a block diagram of one embodiment of an IMD 501 that is implanted into the patient, optionally as part of the implantable cardiac system in accordance with certain embodiments herein. Optionally, the IMD 501 may provide full-function cardiac resynchronization therapy. Alternatively, the IMD 501 may be implemented with a reduced set of functions and components, e.g., if the IMD is an ICM. The IMD 501 in FIG. 5 is an example of the IMD 101 as shown in FIGS. 1, 2a, 2b.

The IMD 501 has a housing 500 to hold the electronic/computing components. Housing 500 (which is often referred to as the "can", "case", "encasing", or "case electrode") may be programmably selected to act as the return electrode for certain stimulus modes. It is also possible that the housing 500, if electrically conductive, can act as one of a pair of electrodes that is used to sense a signal. Housing 500 may further include a connector (not shown) with a plurality of terminals 502, 504, 506, 508, and 510. The terminals may be connected to electrodes (e.g., the electrodes 112 and 114, discussed above) that are located in various locations on housing 500 and/or on a cardiac lead elsewhere within and about the heart. The IMD 501 includes a programmable microcontroller 520 that controls various operations of the IMD 501, including cardiac monitoring and stimulation therapy. Microcontroller 520, which is an example of the controller 120 described above with reference to FIGS. 1, 2A, and 2B, includes a microprocessor (or equivalent control circuitry), RAM and/or ROM memory, logic and timing circuitry, state machine circuitry, and I/O circuitry.

The IMD 501 further includes a pulse generator 522 that generates stimulation pulses for delivery by one or more electrodes coupled thereto. Pulse generator 522 is controlled by microcontroller 520 via control signal 524. Pulse generator 522 may be coupled to the select electrode(s) via an electrode configuration switch 526, which includes multiple switches for connecting the desired electrodes to the appropriate I/O circuits, thereby facilitating electrode programmability. Switch 526 is controlled by a control signal 548 from microcontroller 520.

In the embodiment of FIG. 5, a single pulse generator 522 is illustrated. Optionally, the IMD 501 may include multiple pulse generators, similar to pulse generator 522, where each pulse generator is coupled to one or more electrodes and controlled by microcontroller 520 to deliver select stimulus pulse(s) to the corresponding one or more electrodes.

Microcontroller 520 is illustrated as including timing control circuitry 532 to control the timing of the stimulation pulses (e.g., pacing rate, atrio-ventricular (AV) delay, atrial interconduction (A-A) delay, or ventricular interconduction (V-V) delay, etc.). Timing control circuitry 532 may also be used for the timing of refractory periods, blanking intervals, noise detection windows, evoked response windows, alert intervals, marker channel timing, and so on. Microcontroller 520 also has an arrhythmia detector 534 for detecting arrhythmia conditions. Although not shown, the microcontroller 520 may further include other dedicated circuitry and/or firmware/software components that assist in monitoring various conditions of the patient's heart and managing pacing therapies.

The microcontroller 520 is also shown as including an optional cardiac event and episode discriminator 536, which can use a non-biological disturbance detected by the DDC 210 to distinguish between a true or a false detected cardiac event (e.g., PVC or PAC), and/or to distinguish between a true or a false detected cardiac episode (e.g., asystole or sinus pause). In other words, the cardiac event and episode discriminator 536 can determine, based at least in part on a non-biological disturbance being detected, whether to classify a detected episode of asystole or sinus pause as being a false detection. Additionally, or alternatively, the cardiac event and episode discriminator 536 can determine, based at least in part on the non-biological disturbance being detected, whether to classify a detected PVC or PAC as being a false detection.

The IMD 501 is further equipped with an optional communication modem (modulator/demodulator) 540 to enable wireless communication with other devices. In one implementation, modem 540 may use low or high frequency modulation. As one example, modem 540 may transmit implant-to-implant (i2i) messages and other signals through conductive communication between a pair of electrodes. The modem 540 can alternatively, or additionally, be used to provide RF communication and/or inductive communication. Modem 540 may be implemented in hardware as part of microcontroller 520, or as software/firmware instructions programmed into and executed by microcontroller 520. Alternatively, modem 540 may reside separately from the microcontroller 520 as a standalone component.

The IMD 501 includes a sensing circuit 544 selectively coupled to a pair of electrodes (e.g., 112 and 114), that perform sensing operations, through switch 526 to detect the presence of cardiac activity in the right chambers of the heart. Sensing circuit 544 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. For example, the sensing circuit 544 can include the sense amplifier 116 described above with reference to FIGS. 1, 2A and 2B. The automatic gain control enables the unit to sense low amplitude signal characteristics of atrial fibrillation. Switch 526 determines the sensing polarity of the cardiac signal by selectively closing the appropriate switches. In this way, the clinician may program the sensing polarity independent of the stimulation polarity.

The output of sensing circuit 544 is connected to microcontroller 520 which, in turn, triggers or inhibits the pulse generator 522 in response to the presence or absence of cardiac activity. Sensing circuit 544 receives a control signal 546 from microcontroller 520 for purposes of controlling the gain, threshold, polarization charge removal circuitry (not shown), and the timing of any blocking circuitry (not shown) coupled to the inputs of the sensing circuitry. The output of the sensing circuit 544, or an output of a sub-circuit thereof, can be provided to an embodiment of the non-biological DDC 210, e.g., described above with reference to FIGS. 2A and 2B, in order to enable the IMD 501 to detect a non-biological disturbance, examples of which were discussed above.

In the embodiment of FIG. 5, a single sensing circuit 544 is illustrated. Optionally, the IMD 501 may include multiple sensing circuits, similar to sensing circuit 544, where each sensing circuit is coupled to one or more electrodes and controlled by microcontroller 520 to sense electrical activity detected at the corresponding one or more electrodes. Sensing circuit 544 may operate in a unipolar sensing configuration or in a bipolar sensing configuration.

The IMD 501 further includes an analog-to-digital (A/D) data acquisition system (DAS) 550 coupled to one or more electrodes via switch 526 to sample cardiac signals across any pair of desired electrodes. Data acquisition system 550 is configured to acquire intracardiac electrogram signals, convert the raw analog data into digital data, and store the digital data for later processing and/or telemetric transmission to an external device 554 (e.g., a programmer, local transceiver, or a diagnostic system analyzer). Accordingly, the DAS 550 can be or include an ADC. Data acquisition system 550 is controlled by a control signal 556 from the microcontroller 520.

The signal output by the data acquisition system 550 can be provided to the non-biological DDC 210, e.g., described above with reference to FIGS. 2A and 2B, or a portion thereof. Alternatively, the signal output by the sense circuit 544 can be provided to the non-biological DDC 210, e.g., described above with reference to FIGS. 2A and 2B, or a portion thereof. The IMD 501 of FIG. 5 can thereby be equipped with a non-biological DDC 210 connected to the sense circuit 544, or a non-biological DDC 210 connected to the data acquisition system 550, or a first non-biological DDC 210 connected to the sense circuit 544 and a second non-biological DDC 210 connected to the data acquisition system 550.

Microcontroller 520 is coupled to a memory 560 by a suitable data/address bus. The programmable operating parameters used by microcontroller 520 are stored in memory 560 and used to customize the operation of IMD 501 to suit the needs of a particular patient. Such operating parameters define, for example, pacing pulse amplitude, pulse duration, electrode polarity, rate, sensitivity (e.g., a sense detection threshold or gain), automatic features, arrhythmia detection criteria, and the amplitude, waveshape and vector of each shocking pulse to be delivered to the patient's heart within each respective tier of therapy. Other types of operating parameters that can be stored in the memory 560 include the various thresholds described herein, including the slew rate threshold, the magnitude threshold, and the threshold period of time, but are not limited thereto.

The operating parameters of IMD 501 may be non-invasively programmed into memory 560 through an optional telemetry circuit 564 in telemetric communication via communication link 566 with external device 554. Telemetry circuit 564 allows intracardiac electrograms and status information relating to the operation of IMD 501 (as contained in microcontroller 520 or memory 560) to be sent to external device 554 through communication link 566.

The IMD 501 can optionally include magnet detection circuitry (not shown), coupled to microcontroller 520, to detect when a magnet is placed over the unit. A magnet may be used by a clinician to perform various test functions of IMD 501 and/or to signal microcontroller 520 that external device 554 is in place to receive or transmit data to microcontroller 520 through telemetry circuits 564.

The IMD 501 can optionally include one or more physiological sensors 570. Such sensors are commonly referred to as "rate-responsive" sensors because they are typically used to adjust pacing stimulation rates according to the exercise state of the patient. However, physiological sensor 570 may further be used to detect changes in cardiac output, changes in the physiological condition of the heart, or diurnal changes in activity (e.g., detecting sleep and wake states). Signals generated by physiological sensors 570 are passed to microcontroller 520 for analysis. Microcontroller 520 responds by adjusting the various pacing parameters (such as rate, AV Delay, V-V Delay, etc.) at which the atrial and ventricular pacing pulses are administered. While shown as being included within IMD 501, physiological sensor(s) 570 may be external to IMD 501, yet still be implanted within or carried by the patient. Examples of physiologic sensors might include sensors that, for example, sense respiration rate, pH of blood, ventricular gradient, activity, position/posture, minute ventilation (MV), and so forth.

A battery 572 provides operating power to all of the components in IMD 501. Battery 572 is capable of operating at low current drains for long periods of time, and is capable of providing high-current pulses (for capacitor charging) when the patient requires a shock pulse (e.g., in excess of 2 A, at voltages above 2 V, for periods of 10 seconds or more). Battery 572 also desirably has a predictable discharge characteristic so that elective replacement time can be detected. As one example, IMD 501 employs lithium/silver vanadium oxide batteries.

The IMD 501 optionally includes an impedance measuring circuit 574, which can be used for many things, including: lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. Impedance measuring circuit 574 is coupled to switch 526 so that any desired electrode may be used. In this embodiment IMD 501 further includes a shocking circuit 580 coupled to microcontroller 520 by a data/address bus 582.

An aspect of the present technology relates to an IMD (e.g., 101, 501) configured to be implanted in a patient, wherein the IMD comprises sense circuitry (e.g., 116, 544, 550) and disturbance detection circuitry (e.g. 210). The sense circuitry is configured to produce a differential signal indicative of a voltage potential difference between first and second electrodes. The first and second electrodes can be, e.g., at least two of the electrodes 112, 114, 500, and/or electrodes electrically coupled to terminals 502, 504, 506, 508, 510. The disturbance detection circuitry is configured to detect a non-biological disturbance based at least in part on a slew rate of the differential signal exceeding a slew rate threshold. The IMD can include both of the first and second electrodes, e.g., if the IMD is an ICM. Alternatively, if the IMD is an ICD and the first and second electrodes are located on a lead that is electrically coupled to the IMD, then the IMD is electrically coupled to both of the first and second electrodes. It would also be possible for the IMD to include the first electrode, e.g., if the first electrode is a "can" electrode, and for the second electrode to be located on a lead that is electrically coupled to the IMD. More generally, the IMD includes one or both of the first and second electrodes, is electrically coupled to one or both of the first and second electrodes, or includes one of the first and second electrodes and is electrically coupled to the other one of the first and second electrodes.

In accordance with certain embodiments, the first and second electrodes are intended to be in contact with tissue of the patient within which the IMD is implanted.

In accordance with certain embodiments, the non-biological disturbance is selected from the group consisting of at least one of the first or the second electrodes losing contact with the tissue of the patient within which the IMD is implanted, exposure of the IMD to electromagnetic interference (EMI), and exposure of the IMD to a time-varying gradient magnetic field from a magnetic resonance imaging (MRI) system.

In accordance with certain embodiments, the disturbance detection circuitry is configured to detect the non-biological disturbance also based on a magnitude of the differential signal exceeding a magnitude threshold for at least a threshold period of time.

In accordance with certain embodiments, the differential signal comprises one of an electrocardiogram (ECG) or an electrogram (EGM) indicative of cardiac electrical activity of the patient within which the IMD is implanted.

In accordance with certain embodiments, the first and second electrodes are intended to be in contact with tissue of the patient within which the IMD is implanted; the non-biological disturbance comprises at least one of the first or the second electrodes losing contact with the tissue of the patient within which the IMD is implanted; and the disturbance detection circuitry is configured to detect, based at least in part on the slew rate of the differential signal exceeding the slew rate threshold, when at least one of the first or the second electrodes loses contact with the tissue of the patient within which the IMD is implanted. In accordance with certain such embodiments, the disturbance detection circuitry is configured to detect when at least one of the first or the second electrodes loses contact with the tissue of the patient within which the IMD is implanted, also based on a magnitude of the differential signal exceeding a magnitude threshold for at least a threshold period of time.

In accordance with certain embodiments, the non-biological disturbance comprises exposure of the IMD to electromagnetic interference (EMI) or exposure of the IMD to a time-varying gradient magnetic field from a magnetic resonance imaging (MRI) system; and the disturbance detection circuitry is configured to detect, based at least in part on the slew rate of the differential signal exceeding the slew rate threshold, when the IMD is exposed to EMI or a time-varying gradient magnetic field from an MRI system. In accordance with certain such embodiments, the disturbance detection circuitry is configured to detect when the IMD is exposed to EMI or a time-varying gradient magnetic field from an MRI system, also based on a magnitude of the differential signal exceeding a magnitude threshold for at least a threshold period of time.

In accordance with certain embodiments, the IMD also comprises a controller that is communicatively coupled to the disturbance detection circuitry. The controller can be configured to detect an episode of asystole or sinus pause, and classify the episode of asystole or sinus pause as being a false positive when the non-biological disturbance detected by the disturbance detection circuitry at least partially coincides with the episode of asystole or sinus pause. Alternatively, or additionally, the controller can be configured to detect a premature ventricular contraction (PVC) or premature atrial contraction (PAC), and classify the detected PVC or PAC as being a false detection when the non-biological disturbance detected by the disturbance detection circuitry at least partially coincides with the detected PVC or PAC. Additionally, or alternatively, the controller can be configured to issue a warning or a notification in response to the non-biological disturbance being detected by the disturbance detection circuitry.

In accordance with certain embodiments, the sense circuitry comprises an amplifier having differential inputs electrically coupled to the first and the second electrodes and an ADC (e.g. 118, 550) configured to digitize an output of the amplifier. In certain such embodiments, the disturbance detection circuitry comprises a digital differentiator (e.g., 214) configured to differentiate the output of the ADC, a first digital comparator (e.g., 218a) configured to compare an output of the digital differentiator to a positive slew rate threshold, a second digital comparator (e.g., 220a) configured to compare the output of the digital differentiator (e.g., 214) to a negative slew rate threshold, and an OR gate (e.g., 238) configured to receive outputs of the first and the second digital comparators and provide an indication of when a slew rate of the output of the ADC is greater than the positive slew rate threshold or less than the negative slew rate threshold. In accordance with certain embodiments, the disturbance detection circuitry also comprises a third digital comparator (e.g., 234) configured to compare an output of the ADC to a positive amplitude threshold, a fourth digital comparator (e.g., 236) configured to compare the output of the ADC to a negative amplitude threshold, and a further OR gate (e.g. 238) configured to receive outputs of the third and the fourth digital comparators and provide an indication of when an amplitude of the output of the ADC is greater than the positive amplitude threshold or less than the negative amplitude threshold. In accordance with certain such embodiments, the disturbance detection circuitry further comprises a counter (e.g., 240) configured to receive an output of the further OR gate and periodically increment a value of the counter while of the output of the ADC remains greater than the positive amplitude threshold, or the output of the ADC remains less than the negative amplitude threshold.

In accordance with certain embodiments, the sense circuitry comprises an amplifier (e.g., 116) having differential inputs electrically coupled to the first and second electrodes (e.g., 112, 114, 500, 502, 504, 506, 508, 510), the disturbance detection circuitry (e.g., 210) comprises analog circuitry (e.g., 212b) configured to detect when the slew rate of the differential signal exceeds the slew rate threshold, and the slew rate threshold is specified based on a potential difference between a pair or reference voltages and a period of a signal that is used to control switches (e.g., S1, S2) of a pair of sample and hold circuits of the analog circuitry.

In accordance with certain embodiments, the IMD comprises an ICM configured to be implanted subcutaneously. Alternatively, the IMD can be an ICD or a pacemaker, but is not limited thereto.

Another aspect of the present technology relates to a method for an IMD detecting a non-biological disturbance. The method comprises: producing a differential signal indicative of a voltage potential difference between first and second electrodes; determining whether a slew rate of the differential signal exceeds a slew rate threshold; and detecting a non-biological disturbance based at least in part on determining that the slew rate of the differential signal exceeds the slew rate threshold. In such embodiments, the IMD can include one or both of the first and second electrodes, can be electrically coupled to one or both of the first and second electrodes, or can include one of the first and second electrodes and be electrically coupled to the other one of the first and second electrodes.

In accordance with certain embodiments, the first and second electrodes are intended to be in contact with tissue of the patient within which the IMD is implanted.

In accordance with certain embodiments, the non-biological disturbance is selected from the group consisting of at least one of the first or the second electrodes losing contact with the tissue of the patient within which the IMD is implanted, exposure of the IMD to EMI, and exposure of the IMD to a time-varying gradient magnetic field from an MRI system.

In accordance with certain embodiments, the method further comprises determining whether a magnitude of the differential signal exceeds a magnitude threshold for at least a threshold period of time, wherein the detecting the non-biological disturbance is also based on determining that the magnitude of the differential signal exceeds the magnitude threshold for at least the threshold period of time.

In accordance with certain embodiments, the determining whether the magnitude of the differential signal exceeds the magnitude threshold comprises: comparing an amplitude of the differential signal to a positive amplitude threshold and to a negative amplitude threshold; and in response to the amplitude of the differential signal being greater than the positive amplitude threshold, or being less than the negative amplitude threshold, determining that the magnitude of the differential signal exceeds the magnitude threshold.

In accordance with certain embodiments, the determining whether the magnitude of the differential signal exceeds the magnitude threshold for at least the threshold period of time comprises: periodically incrementing a value of counter while the amplitude of the differential signal remains greater than the positive amplitude threshold, or remains less than the negative amplitude threshold; and in response the value of the counter being equal to or greater than a counter threshold, determining that the magnitude of the differential signal exceeds the magnitude threshold for at least the threshold period of time.

In accordance with certain embodiments, the first and second electrodes are intended to be in contact with tissue of the patient within which the IMD is implanted; and the non-biological disturbance comprises at least one of the first or the second electrodes losing contact with the tissue of the patient within which the IMD is implanted.

In accordance with certain embodiments, the non-biological disturbance comprises exposure of the IMD to EMI or exposure to a time-varying gradient magnetic field from an MRI system.

In accordance with certain embodiments, the differential signal comprises one of an ECG or an EGM indicative of cardiac electrical activity of the patient within which the IMD is implanted.

In accordance with certain embodiments, the method further comprises classifying a detected episode of asystole or sinus pause as being a false positive when the non-biological disturbance at least partially coincides with the episode of asystole or sinus pause; or classifying a detected premature ventricular contraction (PVC) or premature atrial contraction (PAC) as being a false detection when the non-biological disturbance at least partially coincides with the detected PVC or PAC.

In accordance with certain embodiments, the IMD that implements the method comprises an ICM configured to be implanted subcutaneously.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

Embodiments have been described above with the aid of functional building blocks illustrating the performance of specified functions and relationships thereof. The boundaries of these functional building blocks have often been defined herein for the convenience of the description. Alternate boundaries can be defined so long as the specified functions and relationships thereof are appropriately performed. Any such alternate boundaries are thus within the scope and spirit of the claimed invention. For example, it would be possible to combine or separate some of the steps shown in the various flow diagrams. It would also be possible to just perform a subset of the steps shown in the various flow diagrams. For another example, it is possible to change the boundaries of some of the block diagrams.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. An insertable cardiac monitor, ICM, (101, 501) configured to be subcutaneously implanted in a patient, the ICM (101, 501) comprising:

   first and second electrodes (112, 114) intended to be in contact with tissue of the patient within which the ICM (101, 501) is subcutaneously implanted; sense circuitry (116, 544, 550) configured to produce a differential signal indicative of a voltage potential difference between the first and the second electrodes (112, 114); and disturbance detection circuitry (210) configured to detect when at least one of the first or the second electrodes (112, 114) of the ICM loses contact with the tissue of the patient within which the ICM (101, 501) is subcutaneously implanted based at least in part on a slew rate of the differential signal exceeding a slew rate threshold.

2. The ICM (101, 105) of claim 1, wherein one of the first and the second electrodes (112, 114) is an electrically conductive housing (500) of the ICM (101, 105), and the other one of the first and the second electrodes (112, 114) is within a header of the ICM (101, 105).

3. The ICM (101, 501) of any one of claims 1 or 2, wherein the disturbance detection circuitry (210) is configured to detect when the at least one of the first or the second electrodes (112, 114) loses contact with the tissue of the patient within which the ICM (101, 501) is subcutaneously implanted also based on a magnitude of the differential signal exceeding a magnitude threshold for at least a threshold period of time.

4. The ICM (101, 501) of any one of claims 1 through 3, wherein the differential signal comprises an electrocardiogram, ECG, indicative of cardiac electrical activity of the patient within which the ICM (101, 501) is subcutaneously implanted.

5. The ICM (101, 105) of any one of claims 1 through 4, further comprising a controller (120, 520) that is communicatively coupled to the disturbance detection circuitry (210), the controller (120, 520) configured to:

detect an episode of asystole or sinus pause; and

classify the episode of asystole or sinus pause as being a false positive when the detection that at least one of the first or the second electrodes (112, 114) loses contact with the tissue of the patient, detected by the disturbance detection circuitry (210), at least partially coincides with the episode of asystole or sinus pause.

6.  The ICM (101, 105) of any one of claims 1 through 5, further comprising a controller (120, 520) that is communicatively coupled to the disturbance detection circuitry (210), the controller (120, 520) configured to:

detect a premature ventricular contraction, PVC, or premature atrial contraction, PAC; and classify the detected PVC or PAC as being a false detection when the detection that at least one of the first or the second electrodes (112, 114) loses contact with the tissue of the patient, detected by the disturbance detection circuitry (210), at least partially coincides with the detected PVC or PAC.

7.  The ICM (101, 105) of any one of claims 1 through 6, further comprising a controller (120, 520) that is communicatively coupled to the disturbance detection circuitry (210), the controller (120, 520) configured to issue a warning or a notification in response to the disturbance detection circuitry (210) detecting that at least one of the first or the second electrodes (112, 114) loses contact with the tissue of the patient within which the ICM (101, 501) is subcutaneously implanted.

8.  The ICM (101, 105) of any one of claims 1 through 7, wherein:

the sense circuitry (116, 544, 550) comprises an amplifier having differential inputs electrically coupled to the first and the second electrodes (112, 114) and an analog-to-digital converter, ADC, (118) configured to digitize an output of the amplifier; and the disturbance detection circuitry (210) comprises:

a digital differentiator (214) configured to differentiate the output of the ADC (118); a first digital comparator (218a) configured to compare an output of the digital differentiator (214) to a positive slew rate threshold; a second digital comparator (220a) configured to compare the output of the digital differentiator (214) to a negative slew rate threshold; and logic (222, 120) configured to receive outputs of the first and the second digital comparators (218a, 220a) and provide an indication of when a slew rate of the output of the ADC (118) is greater than the positive slew rate threshold or less than the negative slew rate threshold.

9.  The ICM (101, 105) of claim 8, wherein the disturbance detection circuitry (210) further comprises:

a third digital comparator (234) configured to compare an output of the ADC (118) to a positive amplitude threshold; a fourth digital comparator (236) configured to compare the output of the ADC (118) to a negative amplitude threshold; and further logic (238, 120) configured to receive outputs of the third and the fourth digital comparators (234, 236) and provide an indication of when an amplitude of the output of the ADC (118) is greater than the positive amplitude threshold or less than the negative amplitude threshold.

10. The ICM (101, 105) of claim 9, wherein the disturbance detection circuitry (210) further comprises a counter (240, 120) configured to receive an output of the further logic (238, 120) and periodically increment a value of the counter (240, 120) while the output of the ADC (118) remains greater than the positive amplitude threshold, or the output of the ADC (118) remains less than the negative amplitude threshold.

11. The ICM (101, 105) of any one of claims 1 through 10, wherein:

the sense circuitry (116, 544, 550) comprises an amplifier having differential inputs electrically coupled to the first and the second electrodes (112, 114); the disturbance detection circuitry (210) comprises analog circuitry (212b) configured to detect when the slew rate of the differential signal exceeds the slew rate threshold; and the slew rate threshold is specified based on a potential difference between a pair of reference voltages and a period of a signal that is used to control switches (S1, S2) of a pair of sample and hold circuits of the analog circuitry (212b).

**Patentansprüche**

1. Implantierbarer Herzmonitor, ICM, (101, 501), der dazu eingerichtet ist, einem Patienten subkutan implantiert zu werden, wobei der ICM (101, 501) umfasst:

   erste und zweite Elektroden (112, 114), die dazu bestimmt sind, mit Gewebe des Patienten in Kontakt zu sein, dem der ICM (101, 501) subkutan implantiert wurde; eine Erfassungsschaltung (116, 544, 550), die dazu eingerichtet ist, ein Differenzsignal zu erzeugen, das eine Spannungsdifferenz zwischen der ersten und der zweiten Elektrode (112, 114) anzeigt; und
   eine Störungsdetektionsschaltung (210), die dazu eingerichtet ist zu detektieren, wann mindestens eine der ersten oder der zweiten Elektroden (112, 114) des ICM den Kontakt mit dem Gewebe des Patienten verliert, dem der ICM (101, 501) subkutan implantiert wurde, basierend zumindest teilweise darauf, dass eine Anstiegsrate des Differenzsignals einen Anstiegsratenschwellenwert überschreitet.

2. ICM (101, 105) nach Anspruch 1, wobei eine der ersten und der zweiten Elektroden (112, 114) ein elektrisch leitfähiges Gehäuse (500) des ICM (101, 105) ist, und die andere der ersten und der zweiten Elektroden (112, 114) sich innerhalb eines Anschlusskopfs des ICM (101, 105) befindet.

3. ICM (101, 501) nach einem der Ansprüche 1 oder 2, wobei die Störungsdetektionsschaltung (210) dazu eingerichtet ist zu detektieren, wann die mindestens eine der ersten oder der zweiten Elektroden (112, 114) den Kontakt mit dem Gewebe des Patienten, dem der ICM (101, 501) subkutan implantiert wurde, auch basierend darauf verliert, dass ein Betrag des Differenzsignals einen Betragsschwellenwert für mindestens eine Schwellenzeitdauer überschreitet.

4. ICM (101, 501) nach einem der Ansprüche 1 bis 3, wobei das Differenzsignal ein Elektrokardiogramm, EKG, umfasst, das die elektrische Herzaktivität des Patienten anzeigt, dem der ICM (101, 501) subkutan implantiert wurde.

5. ICM (101, 105) nach einem der Ansprüche 1 bis 4, ferner umfassend eine Steuerung (120, 520), die kommunikativ mit der Störungsdetektionsschaltung (210) gekoppelt ist, wobei die Steuerung (120, 520) dazu eingerichtet ist:

   Detektieren einer Episode von Asystolie oder Sinuspause; und
   Klassifizieren der Episode von Asystolie oder Sinuspause als falsch positiv, wenn die Detektion, dass mindestens eine der ersten oder der zweiten Elektroden (112, 114) den Kontakt mit dem Gewebe des Patienten verliert, wie von der Störungsdetektionsschaltung (210) detektiert, zumindest teilweise mit der Episode von Asystolie oder Sinuspause zusammenfällt.

6. ICM (101, 105) nach einem der Ansprüche 1 bis 5, ferner umfassend eine Steuerung (120, 520), die kommunikativ mit der Störungsdetektionsschaltung (210) gekoppelt ist, wobei die Steuerung (120, 520) dazu eingerichtet ist:

   eine vorzeitige ventrikuläre Kontraktion, PVC, oder eine vorzeitige atriale Kontraktion, PAC, zu detektieren; und
   Klassifizieren der detektierten PVC oder PAC als falsche Detektion, wenn die Detektion, dass mindestens eine der ersten oder der zweiten Elektroden (112, 114) den Kontakt mit dem Gewebe des Patienten verliert, wie von der Störungsdetektionsschaltung (210) detektiert, zumindest teilweise mit der detektierten PVC oder PAC zusammenfällt.

7. ICM (101, 105) nach einem der Ansprüche 1 bis 6, ferner umfassend eine Steuerung (120, 520), die kommunikativ mit der Störungsdetektionsschaltung (210) gekoppelt ist, wobei die Steuerung (120, 520) dazu eingerichtet ist, eine Warnung oder eine Benachrichtigung als Reaktion darauf auszugeben, dass die Störungsdetektionsschaltung (210) detektiert, dass mindestens eine der ersten oder der zweiten Elektroden (112, 114) den Kontakt mit dem Gewebe des Patienten verliert, dem der ICM (101, 501) subkutan implantiert wurde.

8. ICM (101, 105) nach einem der Ansprüche 1 bis 7, wobei:

   die Erfassungsschaltung (116, 544, 550) einen Verstärker umfasst, der differenzielle Eingänge aufweist, die elektrisch mit der ersten und der zweiten Elektrode (112, 114) gekoppelt sind, sowie einen Analog-Digital-Wandler, ADC, (118), der dazu eingerichtet ist, einen Ausgang des Verstärkers zu digitalisieren; und
   wobei die Störungsdetektionsschaltung (210) umfasst:

   einen digitalen Differenzierer (214), der dazu eingerichtet ist, den Ausgang des ADC (118) zu differenzieren;
   einen ersten digitalen Komparator (218a), der dazu eingerichtet ist, einen Ausgang des digitalen Differenzierers (214) mit einem positiven Anstiegsratenschwellenwert zu vergleichen;

einen zweiten digitalen Komparator (220a), der dazu eingerichtet ist, den Ausgang des digitalen Differenzierers (214) mit einem negativen Anstiegsratenschwellenwert zu vergleichen; und

eine Logik (222, 120), die dazu eingerichtet ist, Ausgänge des ersten und des zweiten digitalen Komparators (218a, 220a) zu empfangen und eine Anzeige bereitzustellen, wenn eine Anstiegsrate des Ausgangs des ADC (118) größer als der positive Anstiegsratenschwellenwert oder kleiner als der negative Anstiegsratenschwellenwert ist.

9. ICM (101, 105) nach Anspruch 8, wobei die Störungsdetektionsschaltung (210) ferner umfasst:

einen dritten digitalen Komparator (234), der dazu eingerichtet ist, einen Ausgang des ADC (118) mit einem positiven Betragsschwellenwert zu vergleichen;

einen vierten digitalen Komparator (236), der dazu eingerichtet ist, den Ausgang des ADC (118) mit einem negativen Betragsschwellenwert zu vergleichen; und

eine weitere Logik (238, 120), die dazu eingerichtet ist, Ausgänge des dritten und des vierten digitalen Komparators (234, 236) zu empfangen und eine Anzeige bereitzustellen, wenn ein Betrag des Ausgangs des ADC (118) größer als der positive Betragsschwellenwert oder kleiner als der negative Betragsschwellenwert ist.

10. ICM (101, 105) nach Anspruch 9, wobei die Störungsdetektionsschaltung (210) ferner einen Zähler (240, 120) umfasst, der dazu eingerichtet ist, einen Ausgang der weiteren Logik (238, 120) zu empfangen und periodisch einen Wert des Zählers (240, 120) zu inkrementieren, während der Ausgang des ADC (118) größer als der positive Betragsschwellenwert bleibt oder der Ausgang des ADC (118) kleiner als der negative Betragsschwellenwert bleibt.

11. ICM (101, 105) nach einem der Ansprüche 1 bis 10, wobei:

die Erfassungsschaltung (116, 544, 550) einen Verstärker umfasst, der differenzielle Eingänge aufweist, die elektrisch mit den ersten und zweiten Elektroden (112, 114) gekoppelt sind;

die Störungsdetektionsschaltung (210) eine analoge Schaltungsanordnung (212b) umfasst, die dazu eingerichtet ist zu detektieren, wenn die Anstiegsrate des Differenzsignals den Anstiegsratenschwellenwert überschreitet; und

der Anstiegsratenschwellenwert basierend auf einer Potentialdifferenz zwischen einem Paar von Referenzspannungen und einer Periode eines Signals spezifiziert ist, das verwendet wird, um Schalter (S1, S2) eines Paares von Abtasthalteschaltungen der analogen Schaltungsanordnung (212b) zu steuern.

## Revendications

1. Moniteur cardiaque insérable, MCI, (101, 501) configuré pour être implanté par voie sous-cutanée chez un patient, le MCI (101, 501) comprenant :

des première et deuxième électrodes (112, 114) destinées à être en contact avec un tissu du patient à l'intérieur duquel le MCI (101, 501) est implanté par voie sous-cutanée ; une circuiterie de détection (116, 544, 550) configurée pour produire un signal différentiel indicatif d'une différence de potentiel de tension entre la première et la deuxième électrode (112, 114) ; et une circuiterie de détection de perturbation (210) configurée pour détecter quand au moins une parmi la première ou la deuxième électrode (112, 114) du MCI perd le contact avec le tissu du patient à l'intérieur duquel le MCI (101, 501) est implanté par voie sous-cutanée sur la base au moins en partie d'une vitesse de balayage du signal différentiel dépassant un seuil de vitesse de balayage.

2. MCI (101, 105) selon la revendication 1, dans lequel l'une parmi la première et la deuxième électrode (112, 114) est un boîtier électriquement conducteur (500) du MCI (101, 105), et l'autre parmi la première et la deuxième électrode (112, 114) est à l'intérieur d'un connecteur du MCI (101, 105).

3. MCI (101, 501) selon l'une quelconque des revendications 1 ou 2, dans lequel la circuiterie de détection de perturbation (210) est configurée pour détecter quand l'au moins une parmi la première ou la deuxième électrode (112, 114) perd le contact avec le tissu du patient à l'intérieur duquel le MCI (101, 501) est implanté par voie sous-cutanée également sur la base d'une magnitude du signal différentiel dépassant un seuil de magnitude pendant au moins une période de temps seuil.

4. MCI (101, 501) selon l'une quelconque des revendications 1 à 3, dans lequel le signal différentiel comprend un électrocardiogramme, ECG, indicatif d'une activité électrique cardiaque du patient à l'intérieur duquel le MCI (101, 501) est implanté par voie sous-cutanée.

5. MCI (101, 105) selon l'une quelconque des revendications 1 à 4, comprenant en outre un contrôleur (120, 520) qui est couplé de manière communicative à la circuiterie de détection de perturbation (210), le contrôleur (120, 520) étant configuré pour :

> détecter un épisode d'asystolie ou de pause sinusale ; et
>
> classer l'épisode d'asystolie ou de pause sinusale comme étant un faux positif quand la détection qu'au moins une parmi la première ou la deuxième électrode (112, 114) perd le contact avec le tissu du patient, détectée par la circuiterie de détection de perturbation (210), coïncide au moins partiellement avec l'épisode d'asystolie ou de pause sinusale.

**6.** MCI (101, 105) selon l'une quelconque des revendications 1 à 5, comprenant en outre un contrôleur (120, 520) qui est couplé de manière communicative à la circuiterie de détection de perturbation (210), le contrôleur (120, 520) étant configuré pour :

> détecter une contraction ventriculaire prématurée, CVP, ou une contraction auriculaire prématurée, CAP ; et
>
> classer la CVP ou CAP détectée comme étant une fausse détection quand la détection qu'au moins une parmi la première ou la deuxième électrode (112, 114) perd le contact avec le tissu du patient, détectée par la circuiterie de détection de perturbation (210), coïncide au moins partiellement avec la CVP ou CAP détectée.

**7.** MCI (101, 105) selon l'une quelconque des revendications 1 à 6, comprenant en outre un contrôleur (120, 520) qui est couplé de manière communicative à la circuiterie de détection de perturbation (210), le contrôleur (120, 520) étant configuré pour émettre un avertissement ou une notification en réponse à la circuiterie de détection de perturbation (210) détectant qu'au moins une parmi la première ou la deuxième électrode (112, 114) perd le contact avec le tissu du patient à l'intérieur duquel le MCI (101, 501) est implanté par voie sous-cutanée.

**8.** MCI (101, 105) selon l'une quelconque des revendications 1 à 7, dans lequel :

> la circuiterie de détection (116, 544, 550) comprend un amplificateur ayant des entrées différentielles couplées électriquement aux première et deuxième électrodes (112, 114) et un convertisseur analogique-numérique, CAN, (118) configuré pour numériser une sortie de l'amplificateur ; et
>
> la circuiterie de détection de perturbation (210) comprend :
>
> > un différentiateur numérique (214) configuré pour différentier la sortie du CAN (118) ;

> un premier comparateur numérique (218a) configuré pour comparer une sortie du différentiateur numérique (214) à un seuil de vitesse de balayage positif ;
>
> un deuxième comparateur numérique (220a) configuré pour comparer la sortie du différentiateur numérique (214) à un seuil de vitesse de balayage négatif ; et
>
> une logique (222, 120) configurée pour recevoir des sorties des premier et deuxième comparateurs numériques (218a, 220a) et fournir une indication de quand une vitesse de balayage de la sortie du CAN (118) est supérieure au seuil de vitesse de balayage positif ou inférieure au seuil de vitesse de balayage négatif.

**9.** MCI (101, 105) selon la revendication 8, dans lequel la circuiterie de détection de perturbation (210) comprend en outre :

> un troisième comparateur numérique (234) configuré pour comparer une sortie du CAN (118) à un seuil d'amplitude positif ;
>
> un quatrième comparateur numérique (236) configuré pour comparer la sortie du CAN (118) à un seuil d'amplitude négatif ; et
>
> une logique supplémentaire (238, 120) configurée pour recevoir des sorties des troisième et quatrième comparateurs numériques (234, 236) et fournir une indication de quand une amplitude de la sortie du CAN (118) est supérieure au seuil d'amplitude positif ou inférieure au seuil d'amplitude négatif.

**10.** MCI (101, 105) selon la revendication 9, dans lequel la circuiterie de détection de perturbation (210) comprend en outre un compteur (240, 120) configuré pour recevoir une sortie de la logique supplémentaire (238, 120) et incrémenter périodiquement une valeur du compteur (240, 120) tandis que la sortie du CAN (118) reste supérieure au seuil d'amplitude positif, ou que la sortie du CAN (118) reste inférieure au seuil d'amplitude négatif.

**11.** MCI (101, 105) selon l'une quelconque des revendications 1 à 10, dans lequel :

> la circuiterie de détection (116, 544, 550) comprend un amplificateur ayant des entrées différentielles couplées électriquement aux première et deuxième électrodes (112, 114) ;
>
> la circuiterie de détection de perturbation (210) comprend une circuiterie analogique (212b) configurée pour détecter quand la vitesse de balayage du signal différentiel dépasse le seuil de vitesse de balayage ; et

le seuil de vitesse de balayage est spécifié sur la base d'une différence de potentiel entre une paire de tensions de référence et d'une période d'un signal qui est utilisé pour commander des commutateurs (S1, S2) d'une paire de circuits d'échantillonnage et de maintien de la circuiterie analogique (212b).

**FIG. 1**

EP 4 628 153 B1

FIG. 2A

EP 4 628 153 B1

*FIG. 2B*

FIG. 3

EP 4 628 153 B1

```
                          ┌─────────────────────┐ ⌐ 402
                          │ Produce differential│
                          │ signal (e.g., ECG   │
                          │ or EGM) indicative  │
                          │ of voltage potential│
              ┌──────────▶│ difference between  │
              │           │ first and second    │
              │           │ electrodes          │
              │           └──────────┬──────────┘
              │                      │
              │                      ▼           ⌐ 406
              │            ╱───────────────────╲
              │  No       ╱     Slew rate of     ╲
              └──────────⟨  differential signal   ⟩
                          ╲ exceeds slew rate     ╱
                           ╲   threshold?        ╱
                            ╲─────────┬─────────╱
                                      │
                                     Yes
                                      │
                                      ▼           ⌐ 410
                          ┌─────────────────────┐
                          │ Detect non-biological│
                          │ disturbance          │
                          └─────────────────────┘
```

**FIG. 4A**

```
                          ┌─────────────────────┐ ⌐ 402
                          │ Produce differential│
                          │ signal (e.g., ECG   │
                          │ or EGM) indicative  │
                ┌────────▶│ of voltage potential│
                │         │ difference between  │
                │         │ first and second    │
                │         │ electrodes          │
                │         └──────────┬──────────┘
                │                    │
                │                    ▼           ⌐ 406
                │          ╱───────────────────╲
                │  No     ╱     Slew rate of     ╲
                ├────────⟨  differential signal   ⟩
                │         ╲ exceeds slew rate     ╱
                │          ╲   threshold?        ╱
                │           ╲─────────┬─────────╱
                │                     │
                │                    Yes
                │                     │
                │                     ▼           ⌐ 408
                │          ╱───────────────────╲
                │  No     ╱ Magnitude of          ╲
                └────────⟨ differential signal     ⟩
                          ╲ exceeds magnitude      ╱
                           ╲ threshold for at     ╱
                            ╲ least threshold     ╱
                             ╲ period of time?   ╱
                              ╲───────┬────────╱
                                      │
                                     Yes
                                      │
                                      ▼           ⌐ 410
                          ┌─────────────────────┐
                          │ Detect non-biological│
                          │ disturbance          │
                          └─────────────────────┘
```

**FIG. 4B**

**FIG. 5**

**EP 4 628 153 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013253352 A1
- EP 2207593 B1
- US 5910156 A
- US 2016114169 A1
- US 5810739 A
- US 2022111201 A1
- US 2006173295 A1
- US 2017042482 A1